# FASCICULE DE BREVET EUROPEEN

(11) **EP 1 339 616 B1**
(45) Date de publication et mention de la délivrance du brevet: **03.06.2009**
(21) Numéro de dépôt: 01993568.3
(22) Date de dépôt: 13.11.2001
(51) Int. Cl.: B65D 47/18, B65D 51/22, A61F 9/00, A61J 1/14

(54) **DISTRIBUTEUR DE LIQUIDES GOUTTE A GOUTTE**
TROPFENFLÜSSIGKEITSSPENDER
DRIP LIQUID DISPENSER

(30) Priorité: 13.11.2000 FR 0015039
(43) Date de publication de la demande: 03.09.2003
(73) Titulaire: Laboratoires THEA, 63100 Clermont-Ferrand (FR)
(72) Inventeur: FAURIE, Michel, 63960 Veyre-Monton (FR)
(74) Mandataire: Thibon-Littaye, Annick
(86) Numéro de dépôt international: PCT/FR2001/003555
(87) Numéro de publication internationale: WO 2002/038464

(56) Documents cités:
- FR-A- 2 770 495
- US-A- 5 265 770
- US-A- 6 000 578

## Description

La présente invention concerne l'industrie du conditionnement des produits liquides, plus particulièrement la conception et la fabrication de flacons comportant une tête de distribution compte-gouttes incorporée tels que ceux qui servent au conditionnement de liquides à distribuer goutte à goutte.

De tels flacons sont utilisés dans de nombreux domaines d'application, en particulier pour le conditionnement de produits pharmaceutiques ou cosmétiques sous forme liquide, donc notamment sous forme de solutions aqueuses, mais aussi pour des lubrifiants, des colorants, des solutions d'additifs utilisées dans l'industrie alimentaire, etc. Il s'agit de produits liquides que l'on utilise par petites doses, en expulsant à chaque fois quelques gouttes seulement du flacon contenant le liquide, alors que l'on doit conserver le reste pendant longtemps. Bien souvent, le produit conservé dans le flacon doit, en plus, être maintenu à l'abri de toute possibilité de contamination par des agents bactériens ou autres provenant de l'extérieur. Tel est le cas notamment des compositions pharmaceutiques, parmi lesquelles en particulier les solutions ophtalmiques, que l'on prendra ici comme exemple d'application industrielle de l'invention particulièrement pertinent.

Les conditions de pureté exigées du produit empêchent de laisser l'air ambiant rentrer librement dans le flacon pour remplacer en volume la fraction de liquide qui en a été expulsée à chaque opération de distribution. Et s'il est courant d'exiger de ce genre de flacons qu'ils soient équipés d'un moyen dit d'inviolabilité, qui constitue un témoin garantissant qu'ils n'auront pas été ouverts pendant leur période de conservation en magasin avant d'être vendus à un utilisateur final, cette sécurité d'inviolabilité ne joue un rôle que jusqu'à la première utilisation du flacon, si bien qu'elle laisse intact le besoin d'une solution technologique pour préserver la pureté au cours de l'utilisation du flacon par expulsion de doses successives du produit.

Le document US-A-6 000 578 décrit un flacon de distribution de produit goutte à goutte, qui comporte un récipient présentant un col rigide dans lequel se monte une tête de distribution formant un embout extérieur percé d'un canal d'expulsion, et une paroi limitrophe élastiquement déformable pour expulser une fraction du produit à chaque opération de distribution. Ce flacon comporte en outre une cloison fracturable qui est déchirée lors de la première utilisation, par le passage de la tête de distribution d'une position de sécurité à une position de disponibilité.

Le document US-A-5 265 770 décrit un dispositif de distribution de liquide goutte à goutte comportant un réservoir réversiblement déformable et un embout de distribution. Une membrane partiellement hydrophile et partiellement hydrophobe est disposée de manière à empêcher la pénétration de contaminants extérieurs à l'intérieur du réservoir.

Pour répondre aux problèmes que rencontre la pratique industrielle dans le domaine en question, l'invention propose essentiellement de combiner deux moyens de fermeture étanche entre une partie du flacon qui forme récipient contenant le liquide à distribuer et l'extérieur du flacon, l'un d'ordre mécanique, l'autre d'ordre physico-chimique, sous une forme telle que l'on assure une rentrée d'air à chaque étape de distribution du produit tout en évitant les risques de contamination par les agents présents dans l'air extérieur. Les agents contaminants à prendre en considération sont couramment du genre des bactéries, virus et autres agents biologiques, spécialement dans le contexte des produits pharmaceutiques ou cosmétiques, mais il pourra aussi s'agir d'autres formes d'agents nocifs, ceux en particulier qui pourraient entraîner une destruction ou dénaturation du produit à distribuer.

Le flacon suivant l'invention représente un progrès technologique important par rapport aux dispositifs de conditionnement de liquides à distribuer goutte à goutte actuellement connus, dans lesquels on prévoit une déformation manuelle du récipient contenant le liquide à distribuer qui est de type plastique, grâce à une configuration en soufflet cylindrique, ce qui permet de réduire progressivement le volume intérieur au fur et à mesure que le liquide en est expulsé à chaque étape de distribution. Un tel récipient à forme en soufflet doit être protégé par une coque rigide sur laquelle est fixée une tête de distribution qui ferme le col du récipient et se termine hors de ce dernier par un canal d'expulsion des gouttes, la protection contre les contaminations étant obtenue au moyen d'un filtre anti-bactérien qui est disposé dans la tête de distribution en amont du canal d'expulsion des gouttes.

On conçoit aisément que l'on aurait intérêt à pouvoir se dispenser de l'enveloppe supplémentaire du récipient que représente la coque rigide. En fait, on se trouve dans ce cas en présence d'une fabrication complexe et coûteuse, tant du point de vue de la réalisation de chaque pièce constitutive de l'ensemble que de leur assemblage. De plus, si dans le cadre d'une fabrication industrielle, on veut modifier la contenance du flacon en produit, on ne sait y parvenir sans que cela entraîne des modifications importantes de l'installation de production, et par conséquent un coût supplémentaire non négligeable.

Par ailleurs, la structure des flacons connus de ce type impose que le remplissage des flacons soit fait en usine avant la mise en place de la tête de distribution, c'est-à-dire pendant l'assemblage du flacon. L'industriel producteur du liquide à distribuer conditionné dans ces flacons doit donc envoyer sur le lieu de fabrication tous les éléments constitutifs du flacon pour recevoir ensuite les flacons prêts à la vente et se procurer une machine spéciale de remplissage et de montage final du flacon dont le coût est tel qu'elle_ ne peut être rentabilisée que pour de très grandes cadences d'utilisation. Il est donc souhaitable que l'opération de remplissage du flacon puisse être effectuée en dehors de l'usine de fabrication du flacon par l'entreprise qui prépare le liquide à distribuer, par exemple, au moyen d'une machine de remplissage de flacons usuelle.

L'invention vise à répondre aux attentes de l'industrie dans le domaine du conditionnement des liquides à distribuer en gouttes, dont celles qui viennent d'être soulignées. Elle a donc notamment pour but de proposer un nouveau flacon de conditionnement de liquides à distribuer goutte à goutte dont la structure soit simplifiée, qui se prête à un remplissage dans un site automatisé autonome par rapport à la fabrication du flacon, et qui permette d'obtenir des contenances différentes sans grandes modifications de la fabrication.

A cet effet, l'invention a pour objet un flacon de conditionnement pour liquide à distribuer goutte à goutte, du type comportant un récipient terminé par un col rigide dans lequel se monte une tête de distribution formant un embout extérieur percé d'un canal d'expulsion du liquide hors du récipient, qui se **caractérise en ce que** ledit récipient présente une paroi limitrophe élastiquement déformable pour expulser une fraction du liquide contenu dans le réservoir à chaque opération de distribution, en ce que ladite tête de distribution comporte, montée en travers de la circulation du liquide ainsi expulsé du récipient, une membrane partiellement hydrophile pour livrer passage au liquide sous l'effet d'une surpression exercée dans le récipient par déformation de la paroi et partiellement hydrophobe de sorte que la membrane mouillée par le liquide expulsé laisse néanmoins rentrer de l'air extérieur dans le récipient quand ce dernier reprend élastiquement sa forme initiale après chaque opération de distribution par compression manuelle, et en ce qu'il comporte en outre une cloison fracturable solidaire du récipient pour isoler ladite membrane du liquide contenu dans le récipient jusqu'à une première utilisation, ladite cloison étant déchirée par le passage de ladite tête de distribution d'une position de sécurité à une position de disponibilité.

Dans les formes de réalisations préférées du flacon suivant l'invention, en particulier celles qui sont particulièrement destinées au conditionnement de liquides aqueux dans le monde pharmaceutique et cosmétique, et plus spécialement au conditionnement des solutions ophtalmiques, la membrane à la fois hydrophile et hydrophobe est en outre de nature à empêcher l'entrée dans le flacon des agents contaminants présents dans l'atmosphère extérieure. Il s'agit donc d'une membrane dite anti-microbienne qui, suivant ses dimensions de pores, peut arrêter les microbes de plus ou moins grande mobilité.

Suivant d'autres caractéristiques de l'invention, le flacon présente une forme générale cylindrique, ou du moins sensiblement telle (à savoir généralement de section droite circulaire ou oblongue), et sa paroi latérale cylindrique est déformable élastiquement par compression manuelle, en toute ou partie de sa surface, pour assurer la surpression nécessaire à l'expulsion du liquide à chaque distribution. En liaison avec la forme générale cylindrique, on prévoit avantageusement que le fond fermant le récipient à sa partie inférieure, longitudinalement opposée à la cloison fracturable, soit constitué par une capsule rigide qui est fixée étanche sur la paroi cylindrique après le remplissage du récipient.

On se placera désormais ici dans le cadre de tels modes de réalisation de l'invention convenant comme flacon de gouttes ophtalmiques pour compléter la description des caractéristiques de l'invention et de leurs avantages. On soulignera à ce sujet, en considérant un tel flacon dans ses conditions normales de fonctionnement, que la présence d'un compartiment tampon entre la cloison fracturable et la membrane hydrophile / hydrophobe joue un rôle important dans la préservation de la pureté du produit.

L'utilisation d'une membrane à propriétés hydrophile /hydrophobe permet d'utiliser un récipient compressible qui reprend sa forme initiale après chaque opération de distribution par rentrée de l'air pour remplacer le liquide distribué, puisque cet air est rendu stérile par les, propriétés anti-microbiennes de la membrane et que le liquide contenu dans le récipient est ainsi préservé de toute contamination. Cela permet donc de supprimer le soufflet, le récipient étant d'une seule pièce, qui en plus a l'intérêt de pouvoir être globalement cylindrique pour recevoir toutes inscriptions utiles.

L'invention permet de réduire le nombre des éléments constitutifs du flacon et il en résulte une diminution notable de coût de fabrication. De plus, cette structure permet de faire varier la contenance du flacon pour l'adapter aux diverses utilisations avec un faible surcoût d'investissement.

Le fait de prévoir une cloison fracturable qui est solidaire du récipient permet d'utiliser une tête de distribution qui, à la fabrication du flacon, sera mise en place dans le col en haut du récipient, indépendamment du remplissage, lequel s'effectuera au contraire par le bas. De cette manière également, la fabrication du récipient lui-même sera facilitée, et avantageusement réalisée par moulage d'une seule pièce de l'ensemble comprenant sa paroi cylindrique, son col et sa cloison fracturable, du fait que cette cloison transversale intermédiaire sera accessible sur ses deux faces. Il en résulte en outre une réduction du nombre des éléments constituant le flacon, puisque l'on a supprimé la double enveloppe du récipient que constituaient ensemble le soufflet à volume progressivement variable par déformation plastique de sa paroi et la coque extérieure rigide des flacons de l'art antérieur.

Un autre avantage du flacon suivant l'invention réside dans le fait qu'à l'exception de la capsule de fond, ses éléments peuvent être entièrement assemblés en usine avant d'envoyer l'ensemble à un industriel conditionneur qui pourra utiliser une machine de remplissage de type courant dans un atelier de conditionnement automatisé. Pour le remplissage, les flacons sont alors disposés la tête en bas, l'isolation du liquide par rapport à la membrane étant assurée en toute sécurité par la cloison fracturable. Il suffira ensuite de fermer le fond du flacon tant que ce dernier est encore la tête en bas. Cette fermeture s'effectue par fixation de la capsule de fond dont on a déjà parlé, la fixation étanche ayant lieu par toute technique en soi connue, impliquant par exemple soudage, collage et/ou encliquetage. Elle peut aussi s'effectuer, en variante, par collage de la paroi souple du récipient bord à bord suivant un diamètre de sa section transversale.

Selon une autre caractéristique de l'invention, ladite tête de distribution est montée mobile en translation axiale par coulissement dans le col rigide du récipient à paroi élastiquement déformable entre deux positions longitudinales différentes à l'intérieur dudit col, comprenant ladite position de sécurité dans laquelle la communication entre le récipient et ledit canal par l'intermédiaire de ladite membrane est fermée étanche et ladite position de disponibilité où ladite communication est rendue libre par destruction de la cloison fracturable. En combinaison avec cette disposition, la tête de distribution est avantageusement munie, à sa partie inférieure, d'un percuteur apte à fracturer ou déchirer ladite cloison lors d'une première utilisation du récipient impliquant un mouvement d'enfoncement de ladite tête dans ledit col du récipient, en translation longitudinale.

Suivant des caractéristiques secondaires de l'invention, le mouvement de translation ou enfoncement de la tête de distribution peut avantageusement être réalisé, de manière en soi connue, au moyen d'un bouchon venant se visser sur le col rigide du récipient.

D'autre part, la cloison fracturable est de préférence construite de telle sorte qu'elle contribue au contrôle des conditions de distribution du liquide hors du récipient, par exemple en libérant, une fois qu'elle a été percée, une section de passage du liquide qui correspond au moins au quart de la surface active de la membrane hydrophile /hydrophobe.

Pour être fracturable, la cloison comporte avantageusement une pluralité de lignes de faiblesse, ou lignes de prédécoupe, qui sont orientées chacune radialement et disposées en étoile, dans une répartition circulaire régulière, à intervalles angulaires sensiblement égaux. Avantageusement en combinaison avec cette disposition, mais éventuellement séparément, le percuteur est de son côté construit de préférence en respectant une géométrie à symétrie de révolution. C'est ainsi qu'il peut notamment être réalisé sous la forme d'une pluralité d'ailettes radiales triangulaires régulièrement réparties autour de l'axe longitudinal du flacon. Le nombre de lignes de prédécoupe de la cloison est alors avantageusement différent du nombre des ailettes du percuteur. Cette disposition évite que les ailettes les lignes de prédécoupe puisse se trouver toutes en correspondance chacune à chacune, ce qui garantit l'ouverture du récipient par écartement des parois déchirées de la cloison.

Selon encore une autre caractéristique de l'invention, le flacon comporte une bague d'inviolabilité disposée entre le corps intérieur de la tête de distribution et la partie supérieure du col du récipient lorsque la tête de distribution est dans sa position de sécurité. Le fait de disposer cette bague d'inviolabilité entre la tête de distribution et le col du récipient permet d'obtenir un bon positionnement de la tête de distribution dans le col dans la position de sécurité en éliminant tout risque de perforation de la cloison fracturable lors du montage du flacon. La hauteur de la bague matérialise la course de la tête de distribution lors de son enfoncement pour une première utilisation. Après retrait de cette bague, la tête de distribution est alors déplacée très précisément jusqu'à la position dite de disponibilité où la cloison devient ouverte au passage du liquide.

Suivant une variante de réalisation, la bague d'inviolabilité est montée par des languettes cassables solidaires d'un bouchon qui vient se visser en protection de l'embout de distribution. Une telle bague peut alors rester fixe sur le corps du flacon après rupture desdites languettes quand, pour la première utilisation, l'utilisateur force sur le bouchon pour le dévisser.

Cette solution pour les bagues d'inviolabilité est particulièrement avantageuse en combinaison avec d'autres caractéristiques d'un mode de réalisation préféré du dispositif de l'invention qui consiste notamment en des moyens commandant le déplacement de la tête de distribution en translation axiale dans le col du flacon par la rotation du bouchon de protection lors de son dévissage, cette translation provoquant automatiquement l'ouverture de la cloison fracturable pour mettre en comunication le volume intérieur du flacon avec le canal d'expulsion de la tête de distribution par l'intermédiaire d'un compartiment tampon ménagé à l'intérieur de la nacelle formant le corps intérieur de la tête de distribution.

L'invention sera maintenant plus complètement décrite dans le cadre de caractéristiques préférées et de leurs avantages, en faisant référence aux figures 1 à 6 dans lesquelles :
- la figure 1 est une vue en coupe axiale d'un flacon selon l'invention dans lequel la tête de distribution est dans la position de sécurité ;
- la figure 2 est une vue en coupe axiale représentant le récipient et le corps intérieur de la tête de distribution en position de disponibilité ;
- la figure 3 est une vue en coupe suivant la ligne III-III de la figure 1 ;
- la figure 4 est une vue en perspective montrant la partie inférieure du corps intérieur de la tête de distribution avec le percuteur,
- la figure 5 est une vue partielle en coupe axiale représentant la tête de distribution avant son assemblage,
- et la figure 6 illustre en coupe axiale un autre mode de réalisation préféré de l'invention.

On a représenté sur les figures un exemple de réalisation d'un flacon suivant l'invention, tel qu'il est destiné, en exemple de mise en oeuvre de l'invention particulièrement avantageux, au conditionnement de liquides pharmaceutiques devant être distribués goutte à goutte, en particulier de solutions aqueuses ophtalmiques.

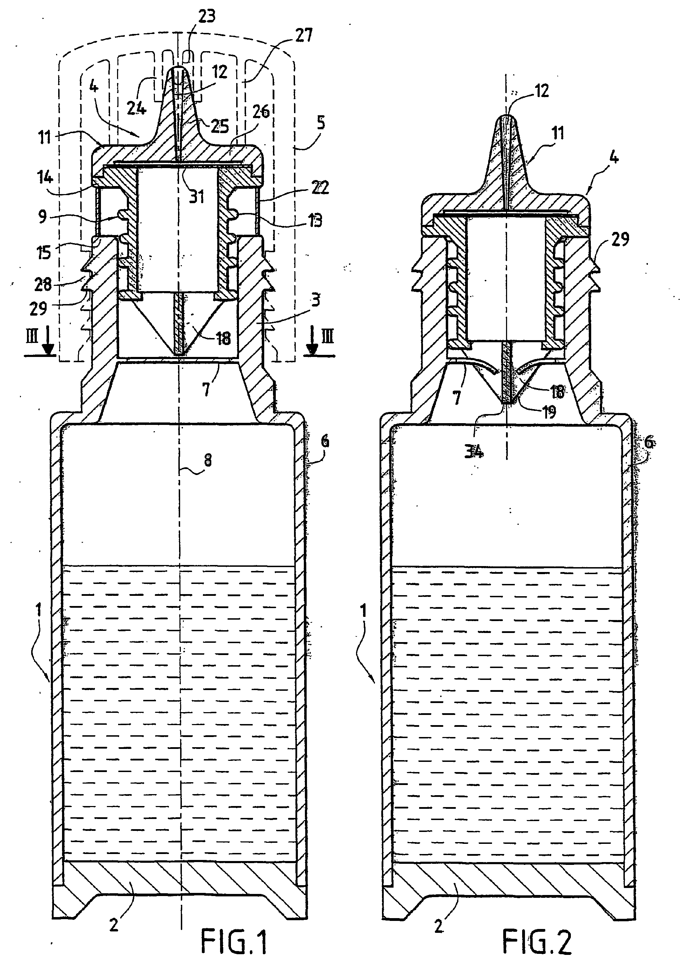

Pour contenir le liquide, le flacon comporte un récipient 1 de forme générale cylindrique, à axe longitudinal vertical sur les figures 1 et 2, qui est fermé à sa partie inférieure par une capsule de fond 2 et qui se prolonge à sa partie supérieure par un col 3 dans lequel se loge une tête de distribution 4, qui y est montée mobile en translation suivant l'axe longitudinal. L'ensemble est complété par un bouchon 5 qui est vissé sur le col 3 du récipient.

Dans toute sa partie latérale cylindrique, la paroi limitrophe 6 du récipient 1 est souple, à déformation élastique, de manière à permettre une réduction temporaire du volume intérieur du récipient lorsque l'on appuie sur cette paroi, le relâchement d'un effort de compression manuelle ayant tendance à ramener le volume intérieur du récipient à sa valeur au repos par retour élastique de sa paroi à la forme cylindrique d'origine. Par contre, la capsule 2 et le col 3 sont rigides.

Le récipient 1 comporte d'autre part une cloison transversale radiale 7, perpendiculaire à son axe longitudinal 8, qui est disposée à la partie inférieure du col 3. Cette cloison intermédiaire est conçue, comme il sera décrit plus loin, pour se déchirer automatiquement lors de la première utilisation du flacon en distributeur de gouttes et créer ainsi une ouverture dans le récipient 1, en amont du col 3.

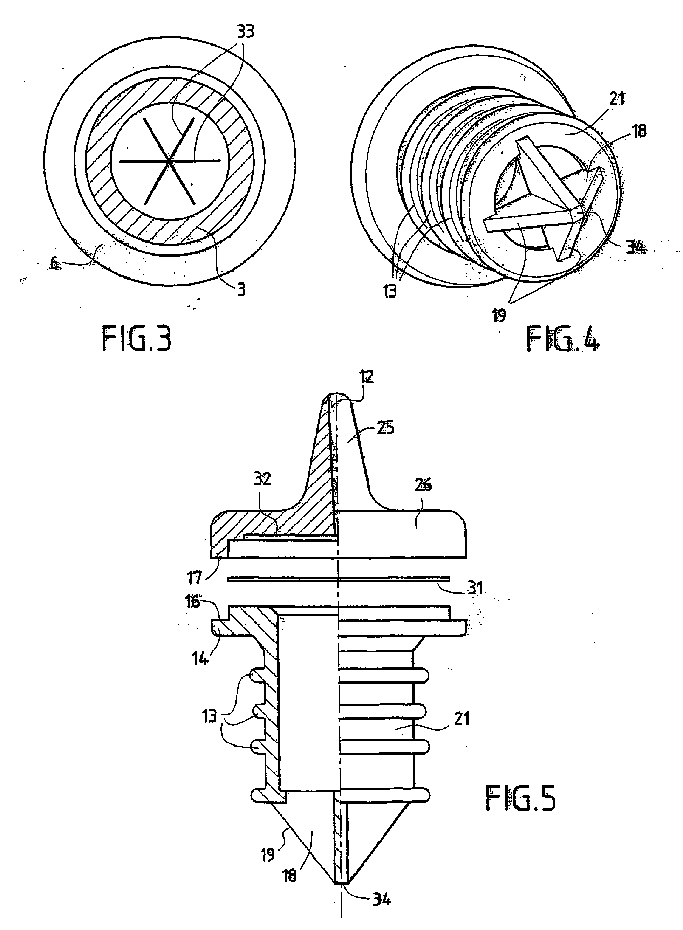

La tête de distribution 4 est réalisée en deux parties solidaires l'une de l'autre, à savoir un corps intérieur ou nacelle 9, qui est de forme annulaire creuse et monté mobile en déplacement axial dans le col 3, et un embout 11 qui est fixé sur la face supérieure de la nacelle 9 et qui est percé d'un canal 12 d'expulsion du liquide hors du flacon. L'étanchéité de la nacelle 9 sur la paroi intérieure du col 3 est assurée au moyen de trois bourrelets annulaires extérieurs 13, de telle sorte qu'il y en ait toujours deux qui soient actifs en étanchéité de la liaison entre la nacelle et le col du récipient. A sa partie supérieure, la nacelle 9 comporte un épaulement 14 qui est destiné à venir en appui sur la face supérieure 15 du col 3 lorsque la tête de distribution 4 est en position de disponibilité comme représentée sur la figure 2. Sur la face supérieure de l'épaulement 14 est aménagé un évidement annulaire 16 dans lequel vient se loger une collerette inférieure 17 de l'embout 11 (figure 5).

A sa partie inférieure, la nacelle 9 comporte un percuteur 18 (voir figures 4 et 5) qui a pour fonction d'assurer l'ouverture de la cloison 7 par fracturation définitive de celle-ci lors de la première utilisation. Ce percuteur est réalisé sous la forme de quatre ailettes radiales 19, forme triangulaire, disposées à angle droit l'une de l'autre, qui se raccordent entre elles dans l'axe du flacon et chacune à la partie inférieure 21 de la nacelle 9. Dans la position de sécurité représentée sur la figure 1, une bague annulaire 22 est intercalée entre la face supérieure 15 du col 3 et la face inférieure de l'épaulement 14 de la nacelle 9. Cette bague joue le rôle classique d'inviolabilité. Elle est rendue solidaire de l'une des deux pièces, ici du col 3 sous une forme telle qu'il est aisé de procéder à son retrait en la déchirant ou la cassant lors de la première utilisation du flacon. En pratique, elle est venue de moulage avec le récipient 1 et son col 3.

Le bouchon 5 comporte un pion central 23 qui s'engage légèrement dans l'extrémité évasée du canal d'expulsion 12, une couronne annulaire 24 qui le guide en centrage sur la face externe d'un prolongement conique 25 de la base 26 de l'embout 11, et une autre couronne annulaire 27, de plus large diamètre, qui vient prendre appui sur la face extérieure de la base 26. Le bouchon 5 comporte un filetage intérieur 28 qui coopère avec un filetage extérieur 29 du col 3.

Conformément à l'invention, et comme on peut le voir en particulier sur la figure 5, une membrane 31 est interposée sur le circuit du liquide expulsé du récipient 1, en travers de la tête de distribution goutte à goutte 4. Elle est disposée maintenue sur son pourtour entre la nacelle 9 et l'embout 11, plus précisément entre la face supérieure de l'épaulement 14 et la face inférieure de la base 26 de l'embout 11.

La face inférieure de la base 26 de l'embout 11 comporte une cuvette circulaire centrale 32 dont le diamètre est supérieur au diamètre intérieur de la nacelle 9 et dans lequel débouche la partie inférieure du canal d'expulsion 12. De cette manière, la membrane 31 n'est fixée que sur sa périphérie entre la nacelle 9 et l'embout 11, et la très large majorité de sa surface est libre et accessible à la circulation du liquide dans l'espace correspondant à l'intérieur de la nacelle 9.

Conformément à l'invention, cette membrane 31 présente, tout d'abord, des propriétés anti-microbiennes qui permettent le filtrage des bactéries et assurent donc que le liquide contenu dans le récipient est maintenu stérile. Par ailleurs, cette membrane est à la fois hydrophile et hydrophobe de telle manière qu'elle permet à la fois la sortie du liquide ophtalmique hors du récipient, la rentrée du liquide expulsé en trop lorsque les gouttes ont été distribuées et la rentrée d'air dans le récipient pour compenser le volume de liquide qui a été distribué sous forme de gouttes.

La tête de distribution est fabriquée de la manière suivante. On réalise donc tout d'abord séparément l'embout 11 et la nacelle 9. On fixe la membrane 31 dans la partie inférieure de l'embout 11 en réalisant une soudure périphérique au moyen d'ultrasons ou de hautes fréquences. La membrane 31 est alors protégée par la collerette 17 pour éviter qu'elle ne subisse des détériorations lors de la manipulation de l'embout 11. L'ensemble de l'embout 11 et de la membrane 31 est ensuite fixé, la collerette 17 de l'embout 11 venant se loger dans l'évidement 16 de la nacelle 9. Cette liaison est rendue étanche par exemple par soudure aux ultrasons ou par fusion thermique. On obtient alors la tête de distribution complète 4 qui sera insérée par la suite, par le haut, dans le col 3 du récipient 1.

Comme on peut le voir sur la figure 3, où l'on reconnaît la partie supérieure de la paroi 6, le col 3 et la cloison fracturable, cette dernière est fragilisée dans sa partie centrale par six lignes de faiblesse, ou lignes de pré-découpe 33, qui sont disposées radialement et réparties régulièrement à écart angulaire de 60 degrés. De cette manière, lorsque l'on exerce une pression au centre de la cloison 7, elle se déchire suivant les lignes 33, et elle ménage ainsi en son centre un conduit de passage pour le liquide contenu dans le récipient 1.

A cet effet, la nacelle 9 comporte, à sa partie inférieure, un percuteur qui est constitué par les quatre ailettes radiales triangulaires 19 dont les extrémités inférieures déterminent une pointe 34 qui vient percuter le centre de la cloison 7 lorsque la tête de distribution 4 est déplacée vers le bas. Comme on peut le remarquer sur les figures, le percuteur 18 de l'exemple décrit comporte quatre ailettes alors que la cloison 7 présente six lignes de déchirure. Ce nombre différent, d'où résulte une périodicité différente dans la répartition en symétrie de révolution, assure que les ailettes ne puissent jamais toutes coïncider avec les lignes de déchirure et que, de ce fait, les ailettes ne risquent pas de venir se loger dans l'espace laissé par les déchirures et obturer le conduit de passage du liquide.

La fabrication du flacon selon l'invention est très simplifiée puisque l'ensemble du récipient, à savoir sa paroi latérale 6, le col 3 avec également la bague d'inviolabilité 22 et la cloison 7 sont réalisés en une seule pièce par une opération d'injection ou d'extrusion de matière synthétique. Il s'agit, par exemple de polyéthylène à haute ou basse pression, mais on peut utiliser tout autre polymère ou matériau composite présentant une barrière suffisante à la vapeur d'eau ainsi qu'une souplesse suffisante (en fonction d'une épaisseur adaptée) pour assurer la fonction de compression que la paroi cylindrique doit remplir. Cette fabrication par moulage d'une seule pièce est facilitée par le fait que la cloison fracturable transversale à la paroi cylindrique est accessible des deux côtés en l'absence du fond.

L'embout et la nacelle de la tête de distribution sont fabriqués séparément, puis la membrane est fixée à l'intérieur de la cuvette 32 par soudure aux ultrasons ou hautes fréquences. L'ensemble de l'embout et de la membrane est ensuite placé sur la nacelle, la collerette 17 venant se loger dans l'évidement 16 de la nacelle. L'embout et la nacelle sont ensuite solidarisés de manière étanche, par exemple par soudure, de manière à constituer la tête de distribution 4.

La tête de distribution 4 est alors engagée dans le col 3 jusqu'à ce qu'elle vienne en butée sur la bague d'inviolabilité dans la position de sécurité où le percuteur 18 se trouve légèrement au-dessus de la cloison 7, comme on peut le voir sur la figure 1. On peut alors visser le bouchon 5 sur le col 3 jusqu'à ce qu'il vienne en butée sur la base 26 de l'embout 11.

L'ensemble ainsi obtenu et la capsule de fond 2 peuvent être envoyés à un fabricant de produits pharmaceutiques commercialisant le liquide à distribuer, qui pourra placer les flacons en position tête en bas dans une machine de remplissage de type connu où le récipient sera rempli, sans risque de contact avec la membrane 31 du fait de la présence de la cloison 7. Lorsque le flacon est rempli, on fixe la capsule de fermeture de fond 2 sur le fond de la paroi 6, de manière étanche, par exemple par soudure, et le flacon rempli de liquide est alors prêt à être commercialisé.

Lors du premier emploi, il suffit de dévisser le bouchon 5, d'ôter la bague d'inviolabilité 22 et de revisser le bouchon 5 sur le col 3. Ce mouvement de vissage permet d'enfoncer la tête de distribution 4 jusqu'à ce qu'elle parvienne dans la position de disponibilité qui est représentée sur la figure 2 après que le percuteur 18 ait causé la déchirure de la cloison 7 selon les lignes de prédécoupe 33. Les sections triangulaires découpées de la cloison 7 sont écartées par les ailettes du percuteur 18 de manière à obtenir une section de passage du liquide contenu dans le récipient 1 correspondant au moins aux trois-quarts de la surface libre de la membrane 31. La partie centrale 34 du percuteur 18 permet d'éviter un jet direct du liquide sur la membrane 31.

Lors de chaque utilisation, on presse sur la paroi 6 du récipient 1, le flacon étant disposé la tête en bas pour obtenir l'éjection d'une ou plusieurs gouttes par le canal 12. Lorsque l'on relâche la pression sur la paroi 6, l'excès de liquide qui a été expulsé hors de la membrane 31 est ré-aspiré dans le flacon grâce aux pores aux propriétés hydrophiles de la membrane 31. Par ailleurs, du fait que cette membrane est mouillée par le liquide, ses pores à propriétés hydrophobes laissent passer de l'air qui vient remplacer le liquide qui a été expulsé sous forme de gouttes. Du fait que la membrane 31 a des propriétés anti-microbiennes, cette réintroduction de liquide et d'air dans le récipient 1 se fait de manière stérile de telle sorte que le liquide contenu dans ce récipient peut être utilisé sur une longue période sans être contaminé.

La membrane peut être réalisée de manière connue en un matériau polymère (à base par exemple de résines de polyamide, de polyvinyle fluoré, de polyéther sulfone, etc) auquel sa composition confère un caractère hydrophile et qui est rendue hydrophobe par modification de sa structure sur une partie de sa surface libre seulement. La modification en question se réalise de manière classique par greffage en présence d'un initiateur de réactions radicalaires. Le traitement est avantageusement effectué pour rendre la membrane hydrophobe suivant une bande diamétrale couvrant de 20 à 50 % de sa surface mouillable par le liquide à expulser. A titre d'exemple, on a ainsi utilisé une membrane à base de résines de polyamide présentant un diamètre de pores de 0,2 micron. Suivant un autre exemple, on a utilisé pour un même débit de passage de la solution aqueuse considérée, une membrane à base de résines de polyéther sulfone présentant un diamètre de pores ramené à 0,1 micron, ce qui permet d'améliorer la filtration anti-microbienne en arrêtant les bactéries les plus mobiles et déformables.

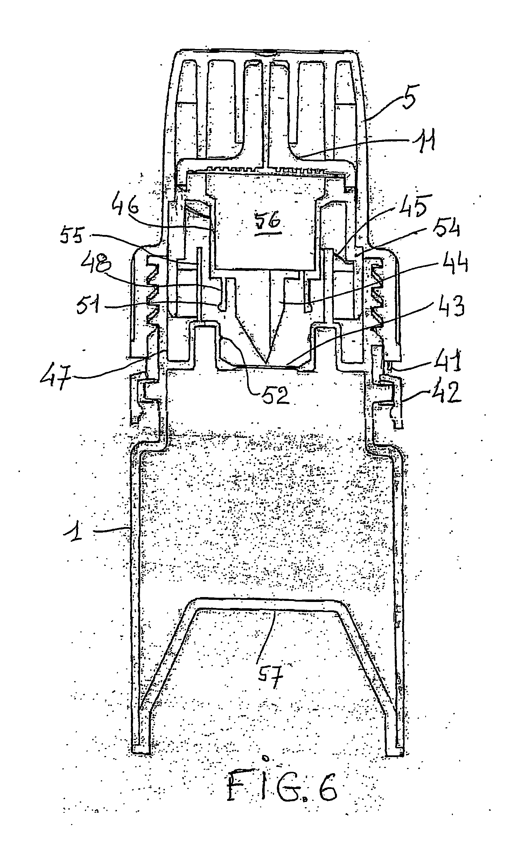

On se reportera maintenant à la figure 6 qui illustre une variante de réalisation du dispositif suivant l'invention qui a l'avantage par rapport aux précédents de faciliter l'utilisation du flacon par le consommateur. En effet, les organes en tête du flacon sont constitués de telle manière que, quand à la première utilisation, le consommateur dévisse le capuchon ou bouchon 5, il commence par provoquer la rupture des languettes 41 qui retiennent la bague d'inviolabilité 42, puis il provoque la perforation de la cloison intermédiaire 43 par le percuteur 44. Quand il continue à dévisser le capuchon 5, il libère celui-ci complètement hors de l'embout distributeur 11.

On observe sur la figure une rampe 45 qui est ménagée à cet effet à l'intérieur de la tête de distribution et qui fait coopérer la nacelle qui constitue son corps intérieur 46 avec des éléments internes du col 47 du flacon. Cette rampe est à filet inverse et de pente nettement plus forte par rapport au filet de vissage du bouchon 5. Elle entre en action quand la nacelle est entraînée en rotation par le bouchon lors du dévissage de ce dernier.

Dans la position illustrée sur la figure, la nacelle 46 est encore en position haute. Elle forme autour du percuteur 44 une couronne 48 qui présente des bossages 51 venant s'encliqueter sur un bourrelet 52 prévu en saillie du corps du flacon, sur la paroi interne d'une coupelle qu'il forme autour de la cloison 43.

Dans sa partie coopérant d'une part avec le filetage du capuchon 5 du côté extérieur, d'autre part avec la nacelle interne 46 par la rampe 45 du côté intérieur, le corps du flacon forme deux couronnes annulaires entre lesquelles vient se placer une jupe externe 54 de la nacelle, cependant qu'une jupe interne de celle-ci terminée par la couronne 48 se place à l'intérieur de la couronne interne 55. La couronne interne se trouve donc intercalée entre les deux jupes co-axiales de la nacelle. C'est à ce niveau qu'est prévue la rampe 45. Cette rampe court sur un demi-tour du flacon, ce qui permet que la partie coopérante de la nacelle vienne en prise avec elle quelle que soit sa position d'origine en rotation.

La figure 6 montre d'autres détails de construction, notamment pour ce qui concerne une came formée sur la jupe 54 en coopération de butée avec un épaulement du bouchon 5 et le fait que la rotation de la nacelle par rapport au col du flacon est limitée à un faible secteur par la présence de cannelures en surépaisseur sur la jupe 54.

On observe aussi sur la figure le compartiment tampon 56 qui, comme dans l'exemple de réalisation illustré par les figures 1 et 2, est ménagé à l'intérieur de la nacelle entre la zone d'entrée formée par des secteurs largement ouverts autour du percuteur 44 et la membrane hydrophile/hydrophobe à l'entrée du canal de distribution de l'embout. Le percuteur 44 est constitué un peu différemment du percuteur 18 de la figure 1. Il forme une pointe de section triangulaire qui est portée en position désaxée par la jupe interne de la nacelle 46.

La figure 6 a l'intérêt d'illustrer l'utilisation de la même tête de distribution et du même corps de flacon de base pour obtenir un flacon global de moindre contenance grâce à un fond bombé 57. Dans ce cas comme dans l'autre, ce fond est fixe sur le flacon pour avoir été soudé dessus lors de son remplissage.

On peut aussi noter que dans le cas de la figure 6, contrairement à celui de la figure 1, la bague d'inviolabilité reste en place sur la bague fixe du corps de flacon sur laquelle elle est montée, mais elle est libérée des languettes cassables qui la fixaient à l'épaulement terminal du bouchon 5.

Il ressort de ce qui précède que l'invention n'est pas limitée aux modes de mise en oeuvre qui ont été spécifiquement décrits dans le courant des exemples ci-dessus et qu'elle s'étend au contraire à toute variante passant par le biais de moyens équivalents. En particulier, on peut utiliser, en tant que percuteur, une canule centrale fixée sur la partie inférieure de la nacelle 9.

Par ailleurs, la capsule de fond peut être fixée par tout moyen permettant une étanchéité suffisante ; on peut par exemple prévoir une fixation par encliquetage. On peut aussi se dispenser de cette capsule en fermant le fond par pincement de la paroi cylindrique du récipient.

L'invention telle qu'elle a été décrite et illustrée ci-dessus n'est pas plus limitée dans ses applications. En particulier, il est évident qu'un dispositif équipé d'une membrane qui, par son caractère hydrophile, est destinée à se laisser traverser par un liquide aqueux, pourra tout aussi bien être utilisée pour la distribution goutte-à-goutte de solutions dans un solvant polaire plutôt que dans l'eau.

## Revendications

1. Flacon de conditionnement pour liquide à distribuer goutte à goutte, comportant un récipient (1) présentant un col rigide (3) dans lequel se monte une tête de distribution (4) formant un embout extérieur (11) percé d'un canal d'expulsion du liquide hors dudit récipient (1), et une paroi limitrophe cylindrique (6) élastiquement déformable pour expulser une fraction du liquide contenu dans le réservoir à chaque opération de distribution, **caractérisé en ce que** ladite paroi déformable est fermée, à sa partie inférieure, par une capsule rigide (2) qui est fixée après le remplissage du récipient (1), **en ce que** ladite tête de distribution comporte, montée en travers de la circulation du liquide ainsi expulsé du récipient, une membrane (31) partiellement hydrophile pour livrer passage au liquide sous l'effet d'une surpression exercée dans le récipient par déformation de la paroi et partiellement hydrophobe de sorte la membrane mouillée par le liquide expulsé laisse néanmoins rentrer de l'air extérieur dans le récipient quand ce dernier reprend élastiquement sa forme initiale, et **en ce qu'**il comporte une cloison fracturable (7) solidaire du récipient qui est disposée de manière à isoler ladite membrane (31) du liquide contenu dans le récipient (1) jusqu'à une première utilisation, ladite cloison étant déchirée par le passage de ladite tête de distribution d'une position de sécurité à une position de disponibilité lors de la première utilisation.

2. Flacon selon la revendication 1, **caractérisé en ce que** ladite membrane (31) est réalisée en un polymère hydrophile, notamment à base de polyamide ou de polyether sulfone, qui est rendu hydrophobe sur une zone couvrant de 20 à 50 % de sa surface et qui présente un diamètre de pores avantageusement de l'ordre de 0,1 ou 0,2 micron, de nature à empêcher l'entrée dans le flacon des agents contaminants présents dans l'atmosphère extérieure.

3. Flacon selon la revendication 1 ou 2, **caractérisé en ce que** ladite paroi du récipient est de forme cylindrique et déformable élastiquement par compression manuelle pour assurer une surpression dans le récipient qui provoque l'expulsion du liquide à chaque opération de distribution, le retour élastique à la forme initiale du récipient provoquant une ré-aspiration du liquide non expulsé hors de l'embout et une rentrée d'air extérieure en compensation du liquide expulsé.

4. Flacon selon la revendication 3, **caractérisé en ce que** ledit récipient est fermé en fond, à l'opposé de ladite tête de distribution par une capsule de fond rigide à fixer à sa paroi cylindrique équipée de la tête de distribution après remplissage du flacon.

5. Flacon selon l'une quelconque des revendications 1 à 4, **caractérisé en ce que** ladite tête de distribution (4) est montée mobile entre deux positions longitudinales différentes à l'intérieur dudit col (3) comprenant ladite position de sécurité dans laquelle la communication entre le récipient (1) et ledit canal d'expulsion (12) par l'intermédiaire de ladite membrane (31) est fermée étanche et ladite position de disponibilité où ladite communication est rendue libre par destruction de la cloison fracturable (7) et **en ce que** ladite tête de distribution (4) est munie, à sa partie inférieure, d'un percuteur (18) apte à fracturer ladite cloison (7) lors d'une première utilisation du récipient impliquant un mouvement d'enfoncement de ladite tête de distribution (4) dans ledit col (3) du récipient.

6. Flacon selon la revendication 5, **caractérisé en ce que** ladite cloison (7) est construite de sorte qu'elle libère alors une section de passage du liquide correspondant au moins au quart de la surface de ladite membrane.

7. Flacon selon la revendication 5 ou 6, **caractérisé en ce que** ladite cloison (7) est réalisée d'une seule pièce en matière moulable avec ledit récipient (1) et ledit col (3).

8. Flacon selon l'une quelconque des revendication 5 à 7, **caractérisé en ce que** la cloison (7) comporte des lignes radiales de prédécoupe (33) disposées en étoile et **en ce que** le percuteur (18) comporte des ailettes radiales (19) également disposées en répartition régulière autour de l'axe longitudinal, le nombre des lignes de prédécoupe (33) de la membrane étant différent du nombre des ailettes (19) du percuteur (18).

9. Flacon selon l'une quelconque des revendications 5 à 8, **caractérisé en ce que qu**'il comporte une bague d'inviolabilité (22) s'intercalant entre un épaulement (14) de la tête de distribution (4) et une face supérieure (15) du col (3) du récipient (1) lorsque la tête de distribution (4) est dans sa position de sécurité, ledit épaulement (14) venant en appui sur ladite face supérieure (15), après retrait de la bague d'inviolabilité, par enfoncement du corps (9) de la tête de distribution (4) jusqu'à la position de disponibilité.

10. Flacon selon l'une quelconque des revendications 5 à 8, **caractérisé en ce qu'**il comporte une bague d'inviolabilité (42) montée à demeure sur le col (3) du récipient (1) qui est relié par des languettes cassables à un bouchon dévissable 5 protégeant la tête de distribution jusqu'à la première utilisation du flacon.

11. Flacon selon l'une quelconque des revendications 5 à 10, **caractérisé en ce qu'**il comporte des moyens à rampe (45) pour provoquer ledit enfoncement de la tête de distribution (4) vers la position de disponibilité lors du dévissage d'un bouchon (5) protégeant ladite tête jusqu'à la première utilisation.

## Claims

1. Bottle for packaging liquid to be dispensed drop by drop, comprising a container (1) having a rigid neck (3) in which a dispensing head (4) is mounted forming an external endpiece (11) perforated by a duct for expelling the liquid from said container (1), and a cylindrical boundary wall (6) which is elastically deformable to expel a fraction of the liquid contained in the reservoir during each dispensing operation, **characterised in that** said deformable wall is closed, at its lower part, by a rigid cap (2) which is fixed after the container (1) has been filled, **in that** said dispensing head comprises a membrane (31), mounted across the flow of liquid thus expelled from the container, which is partially hydrophilic to allow the passage of the liquid by the action of raised pressure exerted in the container by deformation of the wall and which is partially hydrophobic so that the membrane wetted by the expelled liquid nevertheless allows outside air to enter the container when said container elastically reverts to its initial shape, and **in that** it comprises a frangible partition (7) secured to the container which is arranged so as to isolate said membrane (31) from the liquid contained in the container (1) until a first use, said partition being ruptured by the passage of said dispensing head from an inactive position to an active position during the first use.

2. Bottle according to Claim 1, **characterised in that** said membrane (31) is made of a hydrophilic polymer, in particular based on polyamide or polyether sulphone, which is made hydrophobic over a zone covering 20 to 50% of its surface area and which advantageously has a pore diameter in the order of 0.1 or 0.2 microns, capable of preventing contaminants present in the external atmosphere from entering the bottle.

3. Bottle according to Claim 1 or 2, **characterised in that** said wall of the container is of cylindrical shape and elastically deformable by manual compression to provide raised pressure in the container which causes the expulsion of the liquid during each dispensing operation, the elastic return of the container to its initial shape causing unexpelled liquid to be sucked back out of the endpiece and outside air to enter, to compensate for the expelled liquid.

4. Bottle according to Claim 3, **characterised in that** said container is closed at the bottom, opposite said dispensing head, by a cap with a rigid bottom to be fixed to its cylindrical wall provided with the dispensing head, after the bottle has been filled.

5. Bottle according to any one of Claims 1 to 4, **characterised in that** said dispensing head (4) is mounted to be mobile between two different longitudinal positions inside said neck (3), comprising said inactive position in which the communication between the container (1) and said expulsion duct (12) via said membrane (31) is sealingly closed and said active position where said communication is allowed by destroying the frangible partition (7) and **in that** said dispensing head (4) is provided, at its lower part, with a striker (18) capable of breaking said partition (7) during the first use of the container, causing a movement driving said dispensing head (4) into said neck (3) of the container.

6. Bottle according to Claim 5, **characterised in that** said partition (7) is constructed, therefore, so that it opens up a passage cross section for the liquid corresponding to at least one quarter of the surface area of said membrane.

7. Bottle according to Claim 5 or 6, **characterised in that** said partition (7) is produced from a single piece of material which may be moulded with said container (1) and said neck (3).

8. Bottle according to any one of Claims 5 to 7, **characterised in that** the partition (7) comprises precut radial lines (33) arranged in a star configuration and **in that** the striker (18) comprises radial fins (19) also uniformly distributed about the longitudinal axis, the number of precut lines (33) of the membrane being different from the number of fins (19) of the striker (18).

9. Bottle according to any one of Claims 5 to 8, **characterised in that** it comprises a tamper-proof ring (22) inserted between a shoulder (14) of the dispensing head (4) and an upper face (15) of the neck (3) of the container (1) when the dispensing head (4) is in its inactive position, said shoulder (14) coming to bear against said upper face (15), after the removal of the tamper-proof ring, by driving the body (9) of the dispensing head (4) as far as the active position.

10. Bottle according to any one of Claims 5 to 8, **characterised in that** it comprises a tamper-proof ring (42) mounted permanently on the neck (3) of the container (1) which is connected by frangible tabs to an unscrewable stopper 5 protecting the dispensing head until the first use of the bottle.

11. Bottle according to any one of Claims 5 to 10, **characterised in that** it comprises ramp means (45) to induce said driving-in of the dispensing head (4) towards the active position, when unscrewing a stopper (5) which protects said head until the first use.

## Patentansprüche

1. Verpackungsbehältnis zur tropfenweisen Abgabe von Flüssigkeiten (Tropfenflüssigkeitsspender), bestehend aus einem Gefäß (1), das einen starren Hals (3) aufweist, in dem ein Verteilerkopf (4) eingesetzt ist, der ein äußeres Endstück (11) bildet, das von einem Kanal zum Ausbringen der Flüssigkeit aus dem Gefäß (1) durchdrungen ist, und aus einer angrenzenden zylindrischen Wand (6), die elastisch verformbar ist, um bei jedem Abgabevorgang einen Teil der im Behälter befindlichen Flüssigkeit auszubringen, **dadurch gekennzeichnet,**
**dass** die verformbare Wand an ihrem unteren Ende durch einen starren Deckel (2) verschlossen ist, der nach dem Befüllen des Gefäßes (1) befestigt wird,
**dass** der Verteilerkopf eine quer zur Flussrichtung der solchermaßen aus dem Gefäß ausgebrachten Flüssigkeit liegende Membran (31) aufweist, die teilweise hydrophil ist, um den Durchlass der Flüssigkeit unter der Wirkung des im Gefäß durch die Verformung der Wand erzeugten Überdrucks zu ermöglichen, und teilweise hydrophob, so dass die von der ausgebrachten Flüssigkeit benetzte Membran dennoch Außenluft in das Gefäß lässt, wenn dieses wieder elastisch in seine Ausgangsform zurückkehrt,
und **dadurch gekennzeichnet, dass** das Behältnis eine mit dem Gefäß verbundene aufbrechbare Trennwand (7) umfasst, die geeignet ist, die Membran (31) bis zur ersten Nutzung von der Flüssigkeit im Gefäß (1) zu isolieren, wobei die Trennwand durch den Übergang des Verteilerkopfes von einer Sicherungsstellung in eine Bereitschaftsstellung während der ersten Nutzung durchbrochen wird.

2. Behältnis gemäß Anspruch 1, **dadurch gekennzeichnet, dass** die Membran (31) aus einem hydrophilen Polymer besteht, insbesondere auf der Basis von Polyamid oder Polyethersulfon, das über einen Bereich von 20 bis 50% seiner Fläche hinweg hydrophobiert ist und das Poren mit einem Durchmesser vorzugsweise in der Größenordnung von 0,1 oder 0,2 Mikrometern aufweist, so dass der Eintritt von Verunreinigungen aus der Außenatmosphäre in das Behältnis verhindert wird.

3. Behältnis gemäß Anspruch 1 oder 2, **dadurch gekennzeichnet, dass** die Wand des Gefäßes eine zylindrische Form aufweist und durch Druckausübung per Hand elastisch verformbar ist, um im Gefäß einen Überdruck zu erzeugen, der bei jedem Abgabevorgang das Ausbringen von Flüssigkeit bewirkt, wobei die elastische Rückkehr des Gefäßes in die Ausgangsform dazu führt, dass die nicht aus dem Endstück ausgebrachte Flüssigkeit zurückgesaugt wird und zum Ausgleich der ausgebrachten Flüssigkeit Außenluft eintritt.

4. Behältnis gemäß Anspruch 3, **dadurch gekennzeichnet, dass** das Gefäß am Boden - entgegengesetzt zum Verteilerkopf - durch einen steifen Bodendeckel verschlossen ist, der nach dem Befüllen des Behältnisses an seiner mit dem Verteilerkopf ausgestatteten zylindrischen Wand zu befestigen ist.

5. Behältnis gemäß einem der Ansprüche 1 bis 4, **dadurch gekennzeichnet, dass** der Verteilerkopf (4) im Innern des Halses (3) zwischen zwei verschiedenen Stellungen in Längsrichtung beweglich angeordnet ist, nämlich der Sicherungsstellung, bei welcher die Verbindung zwischen dem Gefäß (1) und dem Ausstoßkanal (12) mittels der Membran (31) dicht verschlossen ist, und der Bereitschaftsstellung, bei welcher diese Verbindung durch die Zerstörung der aufbrechbaren Trennwand (7) freigegeben wird,
sowie **dadurch gekennzeichnet, dass** der Verteilerkopf (4) an seinem unteren Ende einen Schlagbolzen (18) aufweist, der geeignet ist, die Trennwand (7) bei der ersten Benutzung des Gefäßes, die eine Absenkbewegung des Verteilerkopfes (4) im Gefäßhals (3) bewirkt, aufzubrechen.

6. Behältnis gemäß Anspruch 5, **dadurch gekennzeichnet, dass** die Trennwand (7) so ausgeführt ist, dass sie in diesem Fall einen Abschnitt für den Durchlass der Flüssigkeit freigibt, der mindestens einem Viertel der Membranfläche entspricht.

7. Behältnis gemäß Anspruch 5 oder 6, **dadurch gekennzeichnet, dass** die Trennwand (7) aus formbarem Material einstückig mit dem Gefäß (1) und dem Hals (3) ausgebildet ist.

8. Behältnis gemäß einem der Ansprüche 5 bis 7, **dadurch gekennzeichnet, dass** die Trennwand (7) mit radialen Teilungslinien (33) versehen ist, die sternförmig angeordnet sind, und dass der Schlagbolzen (18) in ebenfalls regelmäßiger Verteilung um die Längsachse angeordnete radiale Flügel (19) aufweist, wobei sich die Anzahl der Teilungslinien (33) der Membran von der Anzahl der Flügel (19) des Schlagbolzens (18) unterscheidet.

9. Behältnis gemäß einem der Ansprüche 5 bis 8, **dadurch gekennzeichnet, dass** es einen Sicherungsring (22) umfasst, der sich zwischen einer Auskragung (14) des Verteilerkopfes (4) und einer Oberseite (15) des Halses (3) des Gefäßes (1) befindet, wenn der Verteilerkopf (4) in seiner Sicherungsstellung ist, wobei die Auskragung (14) nach dem Entfernen des Sicherungsrings auf der genannten Oberseite (15) zum Aufliegen kommt, und zwar durch Absenken des Rumpfes (9) des Verteilerkopfes (4) in die Bereitschaftsstellung.

10. Behältnis gemäß einem der Ansprüche 5 bis 8, **dadurch gekennzeichnet, dass** es einen Sicherungsring (42) umfasst, der fest am Hals (3) des Gefäßes (1) angebracht ist, der mittels zerbrechbarer Anschlussstücke mit einer abschraubbaren Verschlusskappe (5) zum Schutz des Verteilerkopfes bis zur ersten Nutzung des Behältnisses verbunden ist.

11. Behältnis gemäß einem der Ansprüche 5 bis 10, **dadurch gekennzeichnet, dass** es Rampenmittel (45) umfasst, um das Absenken des Verteilerkopfes (4) in die Bereitschaftsstellung beim Abschrauben der Verschlusskappe (5) zu bewirken, wodurch der Verteilerkopf bis zur ersten Nutzung geschützt bleibt.
